# EUROPEAN PATENT APPLICATION

(11) **EP 3 313 063 A1**
(43) Date of publication of application: **25.04.2018**
(21) Application number: 16811303.3
(22) Date of filing: 11.04.2016
(51) Int. Cl.: H04N 5/369, A61B 1/04, G02B 23/24, G02B 23/26, G03B 17/02

(54) **IMAGING UNIT AND ENDOSCOPE**

(30) Priority: 19.06.2015 JP 2015124066
(71) Applicant: Olympus Corporation, Hachioji-shi Tokyo 192-8507 (JP)
(72) Inventor: ADACHI, Satoru, Tokyo 192-8507 (JP); FUJIMORI, Noriyuki, Tokyo 192-8507 (JP); IGARASHI, Takatoshi, Tokyo 192-8507 (JP)
(74) Representative: Gunzelmann, Rainer
(86) International application number: PCT/JP2016/061735
(87) International publication number: WO 2016/203828

(57) **Abstract**

Provided are an imaging unit and an endoscope which can realize further miniaturization. An imaging unit 40 includes: an image sensor 44 that generates an image signal by receiving light and performing photoelectric conversion; and a relay member 46 that includes silicon substrates 461, 462, and 463 provided on a back surface side of the image sensor 44 opposite to a light receiving surface of the image sensor 44, planar type electronic devices 4611, 4621, and 4631 being formed on the silicon substrates 461, 462, and 463, and relays the image sensor 44 and a signal cable 48 that transmits the image signal.

## Description

### Field

The present invention relates to an imaging unit which is inserted into a subject to capture the inside of a body in the subject and generate image data in the body, and an endoscope having the imaging unit at a distal end portion.

### Background

Conventionally, an endoscope acquires an in-vivo image in a subject such as a patient by inserting, into the subject, a flexible insertion portion having an elongated shape provided with an imaging device at a distal end. An imaging unit used in such an endoscope includes a semiconductor chip on which an image sensor is formed, and a circuit board which is disposed adjacent to a back surface side of the semiconductor chip and on which electronic components such as a capacitor, a resistor, and an IC chip that constitute a driving circuit of the image sensor are mounted (see Patent Literatures 1 and 2).

### Citation List

### Patent Literature

Patent Literature 1: Japanese Patent No. 4575698
Patent Literature 2: Japanese Patent No. 4441305 Summary

### Technical Problem

However, in the above-mentioned Patent Literatures 1 and 2, since the electronic components such as the capacitor, the resistor and the IC chip are provided in an extending direction of a signal cable from a back surface of the semiconductor chip, the imaging unit is vertically long. Therefore, in order to further reduce a burden on a patient, further miniaturization was required.

The present invention has been made in view of the above, and an object thereof is to provide an imaging unit and an endoscope which can realize further miniaturization.

### Solution to Problem

To solve the above-described problem and achieve the object, an imaging unit according to the present invention includes: an image sensor configured to generate an image signal by receiving light and performing photoelectric conversion; and a relay member including a silicon substrate provided on a back surface side of the image sensor opposite to a light receiving surface of the image sensor, a planar type electronic device being formed on the silicon substrate, and relay the image sensor and a signal cable that transmits the image signal.

Moreover, in the above-described imaging unit according to the present invention, a plurality of silicon substrates are laminated in the relay member.

Moreover, in the above-described imaging unit according to the present invention, the plurality of silicon substrates are laminated in a direction orthogonal to an extending direction of the signal cable.

Moreover, in the above-described imaging unit according to the present invention, the relay member further includes a flexible printed board that extends in parallel to an extending direction of the signal cable, and the plurality of silicon substrates are laminated on the flexible printed board.

Moreover, in the above-described imaging unit according to the present invention, the plurality of silicon substrates are laminated in a direction parallel to an extending direction of the signal cable.

Moreover, in the above-described imaging unit according to the present invention, an area of each of the plurality of silicon substrates is equal to or less than a projected area when the image sensor is projected in the extending direction of the signal cable.

Moreover, in the above-described imaging unit according to the present invention, the plurality of silicon substrates are connected by a through via that passes through at least an adjacent silicon substrate.

Moreover, in the above-described imaging unit according to the present invention, the electronic device is formed on each one of both surfaces of each of the plurality of silicon substrates.

Moreover, in the above-described imaging unit according to the present invention, the electronic device is at least any one of a buffer, a capacitor, an inductor and a resistor.

Moreover, an endoscope according to the present invention includes the imaging unit according to the above-described invention; and an insertion portion that includes a cylindrical distal end portion formed of a hard member and is insertable into a subject, wherein the insertion portion includes the imaging unit in an internal space of the distal end portion.

### Advantageous Effects of Invention

According to the present invention, an effect of realizing further miniaturization can be achieved.

### Brief Description of Drawings

FIG. 1 is a diagram schematically illustrating an overall configuration of an endoscope system according to a first embodiment of the present invention.
FIG. 2 is a partial cross-sectional view of a distal end portion of an endoscope according to the first embodiment of the present invention.
FIG. 3 is a cross-sectional view of an imaging unit according to the first embodiment of the present invention.
FIG. 4 is a cross-sectional view of an imaging unit according to a second embodiment of the present invention.
FIG. 5 is a cross-sectional view of a relay member according to a first modification of the embodiments of the present invention.
FIG. 6 is a cross-sectional view of a relay member according to a second modification of the embodiments of the present invention.
FIG. 7 is a schematic cross-sectional view of an imaging unit according to a third modification of the embodiments of the present invention.
FIG. 8 is a schematic cross-sectional view of another imaging unit according to the third modification of the embodiments of the present invention.
FIG. 9 is a schematic cross-sectional view of an imaging unit according to a fourth modification of the embodiments of the present invention.
FIG. 10 is a schematic cross-sectional view of another imaging unit according to the fourth modification of the embodiments of the present invention.

### Description of Embodiments

Hereinafter, an endoscope including an imaging device will be described as a mode for carrying out the present invention (hereinafter referred to as an "embodiment"). In addition, the present invention is not limited by the embodiment. Further, each drawing referred to in the following description only schematically illustrates a shape, a size, and a positional relationship to the extent that contents of the present invention can be understood. That is, the present invention is not limited only to the shape, the size and the positional relationship exemplified in each drawing. Furthermore, dimensions and ratios may be differently illustrated among the drawings.

### (First Embodiment)

### [Configuration of Endoscope System]

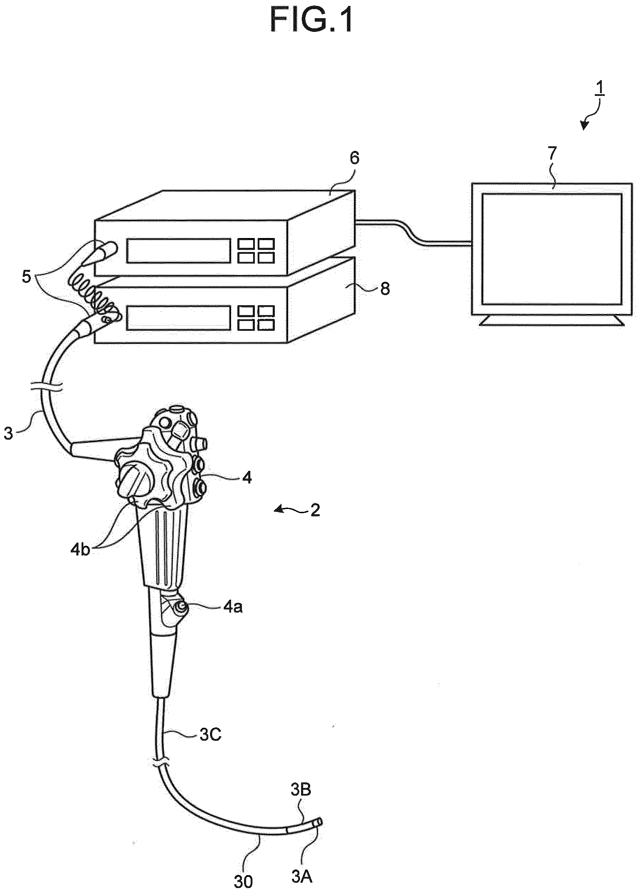

FIG. 1 is a diagram schematically illustrating an overall configuration of an endoscope system according to a first embodiment of the present invention. An endoscope system 1 illustrated in FIG. 1 includes an endoscope 2, a universal cord 3 (transmission cable), a connector portion 5, a processor 6 (control device), a display device 7, and a light source device 8.

The endoscope 2 captures an in-vivo image of a subject and outputs an image signal (image data) to the processor 6 by inserting an insertion portion 30 into the subject. A bundle of electric cables inside the universal cord 3 extends to the insertion portion 30 of the endoscope 2 and is connected to the imaging device provided at a distal end portion 3A of the insertion portion 30. An operating unit 4 provided with various buttons and knobs for operating an endoscope function is connected to a proximal end side of the insertion portion 30 of the endoscope 2. The operating unit 4 has a treatment instrument insertion port 4a for inserting a treatment instrument such as a biological forceps, an electric scalpel, and a test probe in a body cavity of the subject.

The connector portion 5 is provided at a proximal end of the universal cord 3, and is connected to the processor 6 and the light source device 8. The connector portion 5 performs predetermined signal processing on the image signal output from the imaging device of the distal end portion 3A connected to the universal cord 3, performs A/D conversion on the image signal, and outputs a digital image signal to the processor 6.

The processor 6 performs predetermined image processing on the image signal output from the connector portion 5 and outputs the image signal to the display device 7. In addition, the processor 6 controls the entire endoscope system 1. The processor 6 is configured by use of a central processing unit (CPU) or the like.

The display device 7 displays an image corresponding to the image signal output from the processor 6. The display device 7 is configured by use of a display panel such as a liquid crystal display panel or an organic electro luminescence (EL) display panel, and the like.

The light source device 8 irradiates an object with illumination light from the distal end of the insertion portion 30 of the endoscope 2 via the connector portion 5 and the universal cord 3. The light source device 8 is configured by use of a xenon lamp, a light emitting diode (LED) lamp, or the like.

The insertion portion 30 includes the distal end portion 3A provided with the imaging device, a bending portion 3B which is connected to a proximal end side of the distal end portion 3A and freely bendable in a plurality of directions, and a flexible tube portion 3C connected to a proximal end side of the bending portion 3B. The image signal captured by the imaging device provided at the distal end portion 3A is connected to the connector portion 5 via the operating unit 4 by the universal cord 3 having a length of several meters, for example. The bending portion 3B is bent by operation of a bending operation knob 4b provided on the operating unit 4, and is freely bendable, for example in four directions of upward, downward, rightward, and leftward, in accordance with towing and relaxing of bending wire inserted into the insertion portion 30.

In addition, the endoscope 2 is provided with a light guide (not illustrated) for propagating the illumination light from the light source device 8, and is provided with an illumination lens (not illustrated) at an exit end of the illumination light by the light guide. The illumination lens is provided at the distal end portion 3A of the insertion portion 30.

### [Configuration of Distal End Portion of Endoscope]

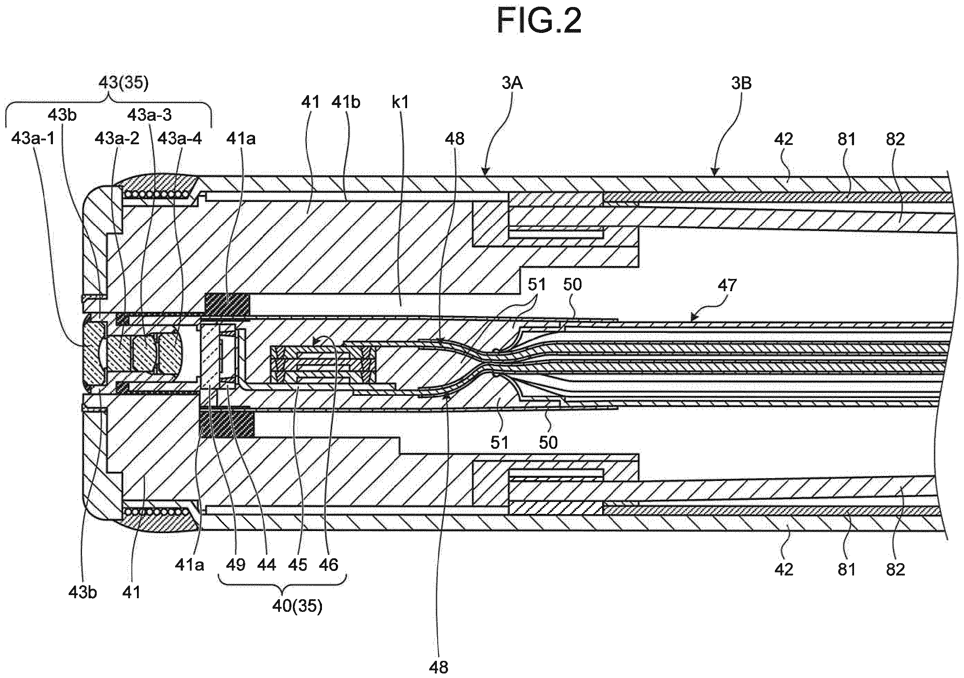

Next, a configuration of the distal end portion 3A of the endoscope 2 will be described in detail. FIG. 2 is a partial cross-sectional view of the distal end portion 3A of the endoscope 2, in a case where the distal end portion 3A is cut at a plane which is orthogonal to a substrate surface of the imaging device provided at the distal end portion 3A of the endoscope 2, and is parallel to an optical axis direction of the imaging device. In addition, FIG. 2 illustrates a part of the distal end portion 3A and the bending portion 3B of the insertion portion 30 of the endoscope 2.

As illustrated in FIG. 2, the bending portion 3B is freely bendable in four directions of upward, downward, rightward, and leftward, in accordance with towing and relaxing of bending wire 82 inserted into a bending tube 81 provided inside a cladding tube 42 described later. An imaging device 35 is provided inside the distal end portion 3A which extends from a distal end side of the bending portion 3B.

The imaging device 35 includes a lens unit 43 and an imaging unit 40 disposed on a proximal end side of the lens unit 43. The imaging device 35 is adhered to the inside of a distal end portion main body 41 by an adhesive 41a. The distal end portion main body 41 is formed into a cylindrical shape by a hard member or the like for forming an internal space k1 that accommodates the imaging device 35. A proximal end side outer peripheral portion 41b of the distal end portion main body 41 is covered with the flexible cladding tube 42. Members closer to the proximal end side than the distal end portion main body 41 are formed of flexible members such that the bending portion 3B is bendable. The distal end portion 3A where the distal end portion main body 41 is disposed becomes a hard portion of the insertion portion 30.

The lens unit 43 includes a plurality of objective lenses 43a-1 to 43a-4 and a lens holder 43b that holds the plurality of objective lenses 43a-1 to 43a-4. The lens unit 43 is fixed to the distal end portion main body 41 by insertion and fixation of a distal end of the lens holder 43b inside the distal end portion main body 41. The plurality of objective lenses 43a-1 to 43a-4 forms an object image.

The imaging unit 40 includes an image sensor 44 such as a charge coupled device (CCD) or a complementary metal oxide semiconductor (CMOS), the image sensor 44 having a light receiving unit that generates an electric signal (image signal) by performing photoelectric conversion by receiving light, a flexible printed board 45 (hereinafter referred to as a "FPC substrate 45") that extends in the optical axis direction from a back side of a light receiving surface of the image sensor 44, a relay member 46 (interposer) made of a silicon substrate which is laminated on a surface of the FPC substrate 45 and on which a planar type electronic device is formed, the relay member 46 relaying the image sensor 44 and each signal cable 48 of an electric cable bundle 47, and a glass slid 49 that adheres to the image sensor 44 while covering the light receiving surface of the image sensor 44. The detailed configuration of the imaging unit 40 will be described later.

A proximal end of the signal cable 48 extends in a proximal end direction of the insertion portion 30. The electric cable bundle 47 is disposed in the distal end portion main body 41 so as to be insertable into the insertion portion 30, and extends to the connector portion 5 via the operating unit 4 and the universal cord 3 illustrated in FIG. 1.

The object image formed by the objective lenses 43a-1 to 43a-4 of the lens unit 43 is received and photoelectrically converted by the image sensor 44 disposed at an image formation position of the objective lenses 43a-1 to 43a-4, and converted into an image signal. The image signal generated by the image sensor 44 is output to the processor 6 via the FPC substrate 45, the relay member 46, the signal cable 48 connected to the relay member 46, and the connector portion 5.

An outer periphery of the imaging device 35 and an outer periphery of a distal end portion of the electric cable bundle 47 are covered with a heat shrinkable tube 50 in order to improve resistance. In the interior of the heat shrinkable tube 50, gaps among parts are filled with a sealing resin 51.

### [Detailed Configuration of Imaging Unit]

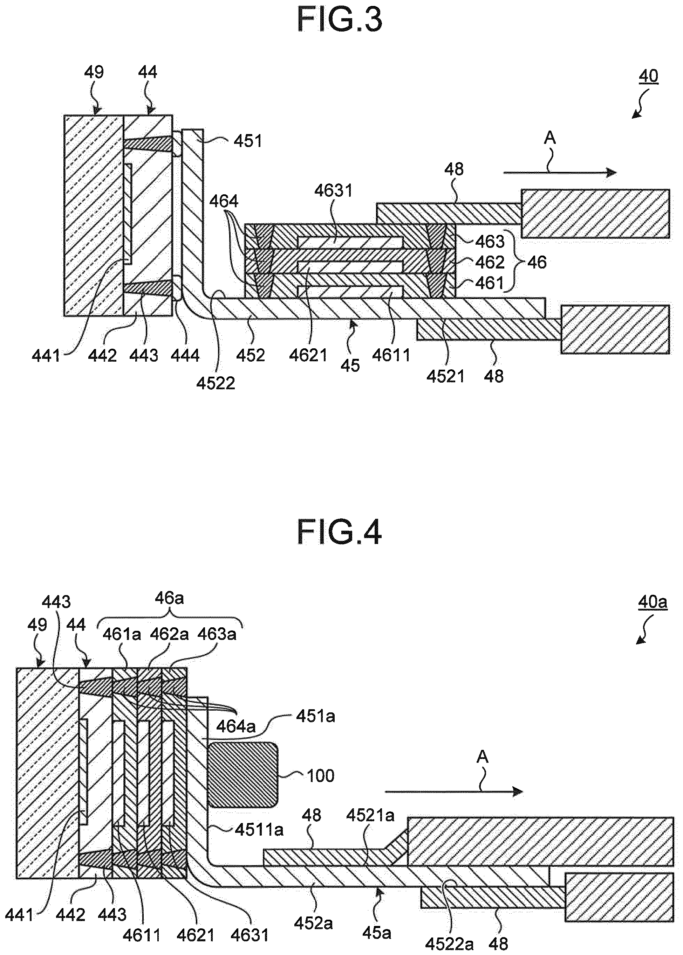

Next, the imaging unit 40 will be described in detail. FIG. 3 is a cross-sectional view of the imaging unit 40. The imaging unit 40 illustrated in FIG. 3 includes the above-mentioned glass slid 49, the image sensor 44, the FPC substrate 45, and the relay member 46.

The image sensor 44 includes a light receiving unit 441 that receives an object image formed by the objective lenses 43a-1 to 43a-4 of the lens unit 43 and performs photoelectric conversion to generate an image signal, a semiconductor substrate 442 on which the light receiving unit 441 is formed, a through via 443 (TSV: Through-Silicon Via) which is provided in the semiconductor substrate 442 and propagates the image signal generated by the light receiving unit 441, and a bump 444 that connects the through via 443 and the FPC substrate 45.

The FPC substrate 45 includes a first substrate 451 connected to a back side of the image sensor 44 via the bump 444, and a second substrate 452 that is continuously extended from one end of the first substrate 451 in a proximal end direction (an extending direction of the signal cable 48) and bent, the proximal end direction being orthogonal to the first substrate 451. In addition, the signal cable 48 is connected to a back surface 4521 of the second substrate 452.

The relay member 46 is provided on a back surface side of the image sensor 44 opposite to the light receiving unit 441 of the image sensor 44, and the relay member 46 includes a plurality of silicon substrates 461 to 463 (semiconductor layers) laminated on a surface 4522 of the second substrate 452 of the FPC substrate 45. The plurality of silicon substrates 461 to 463 has a plurality of electronic devices 4611, 4621, and 4631 formed with a planar type, respectively. The plurality of electronic devices 4611, 4621, and 4631 is laminated in a direction orthogonal (vertical direction) to the extending direction of the signal cable 48 (see arrow A). Each of the electronic devices 4611, 4621, and 4631 is connected by at least an adjacent silicon substrate, in particular via through vias 464 that pass through each layer. The plurality of electronic devices 4611, 4621, and 4631 is any one of a buffer, a capacitor, an inductor, and a resistor that amplify the image signal generated by the image sensor 44 and output the amplified image signal to the signal cable 48. The signal cable 48 is connected to an upper surface of the silicon substrate 463.

According to the first embodiment of the present invention described above, the relay member 46 is provided on the back surface side of the image sensor 44 with respect to the light receiving unit 441, includes the silicon substrates 461 to 463 in which the plurality of electronic devices 4611, 4621, and 4631 is formed with a planar type, and relays the image sensor 44 and the signal cable 48. With this configuration, further miniaturization of the imaging unit 40 can be realized.

In addition, according to the first embodiment of the present invention, further miniaturization of the imaging unit 40 can be realized by lamination of the plurality of silicon substrates 461 to 463 by the relay member 46.

Further, according to the first embodiment of the present invention, the plurality of silicon substrates 461 to 463 is laminated in a direction parallel to the extending direction of the signal cable 48, and the signal cable 48 is connected to the relay member 46. With this configuration, further miniaturization of the imaging unit 40 can be realized.

Furthermore, according to the first embodiment of the present invention, the plurality of silicon substrates 461 to 463 is laminated on the FPC substrate 45, and the signal cable 48 is connected to the back side of the FPC substrate 45. This configuration enables a design with flexibility.

Further, according to the first embodiment of the present invention, since the plurality of silicon substrates 461 to 463 is provided in the internal space of the distal end portion main body 41 of the insertion portion 30 in the endoscope 2, miniaturization of the distal end portion 3A of the endoscope 2 can be realized.

### (Second Embodiment)

Next, a second embodiment of the present invention will be described. The present second embodiment differs from the above-mentioned first embodiment only in the imaging unit 40 according to the first embodiment. Hereinafter, an imaging unit according to the present second embodiment will be described. Note that the same configurations as those of the endoscope system 1 according to the above-mentioned first embodiment are denoted by the same reference numerals, and the description thereof is omitted.

### [Detailed Configuration of Imaging Unit]

FIG. 4 is a cross-sectional view of the imaging unit according to the second embodiment of the present invention. An imaging unit 40a illustrated in FIG. 4 includes the glass slid 49, the image sensor 44, an FPC substrate 45a, a relay member 46a, and a passive element 100.

The relay member 46a includes a plurality of silicon substrates 461a to 463a laminated on the back surface side of the image sensor 44 opposite to the light receiving unit 441 of the image sensor 44. The plurality of silicon substrates 461a to 463a includes the plurality of electronic devices 4611, 4621, and 4631 formed with a planar type, respectively. The plurality of silicon substrates 461a to 463a is laminated on the back surface side of the image sensor 44 such that each area of the plurality of silicon substrates 461a to 463a is equal to or smaller than a projected area when the image sensor 44 is projected in the extending direction of the signal cable 48. The plurality of silicon substrates 461a to 463a is laminated in a direction parallel to the extending direction of the signal cable 48 (see arrow A). Each of the plurality of silicon substrates 461a to 463a is connected by through vias 464a that pass through each layer.

The FPC substrate 45a includes a first substrate 451a connected to a back side of the relay member 46a via a bump (not illustrated), and a second substrate 452a that is continuously extended from one end of the first substrate 451a in a proximal end direction (the extending direction of the signal cable 48) and bent, the proximal end direction being orthogonal to the first substrate 451a.
The signal cable 48 is connected to each of both surfaces 4521a and 4522a of the second substrate 452a of the FPC substrate 45a.

The passive element 100 is connected to a back surface 4511a side of the first substrate 451a of the FPC substrate 45a. The passive element 100 is at least one of a chip capacitor, an inductor, and a resistor.

According to the second embodiment of the present invention as described above, the plurality of silicon substrates 461a to 463a is laminated in the direction parallel to the extending direction of the signal cable 48 (see arrow A). With this configuration, further miniaturization of the imaging unit 40a can be realized.

Furthermore, according to the second embodiment of the present invention, the plurality of silicon substrates 461a to 463a is laminated on the back surface side of the image sensor 44 such that each area of the plurality of silicon substrates 461a to 463a is equal to or smaller than the projected area of the image sensor 44. With this configuration, further miniaturization of the imaging unit 40a can be realized.

### (First Modification)

Next, a first modification of the embodiments of the present invention will be described. The present first modification of the embodiments differs from the above-mentioned first embodiment only in a configuration of the relay member 46 according to the first embodiment. Specifically, a relay member according to the present first modification of the embodiments forms planar type electronic devices on both surfaces of each of the plurality of laminated silicon substrates. Hereinafter, a configuration of the relay member according to the present first modification of the embodiments will be described.

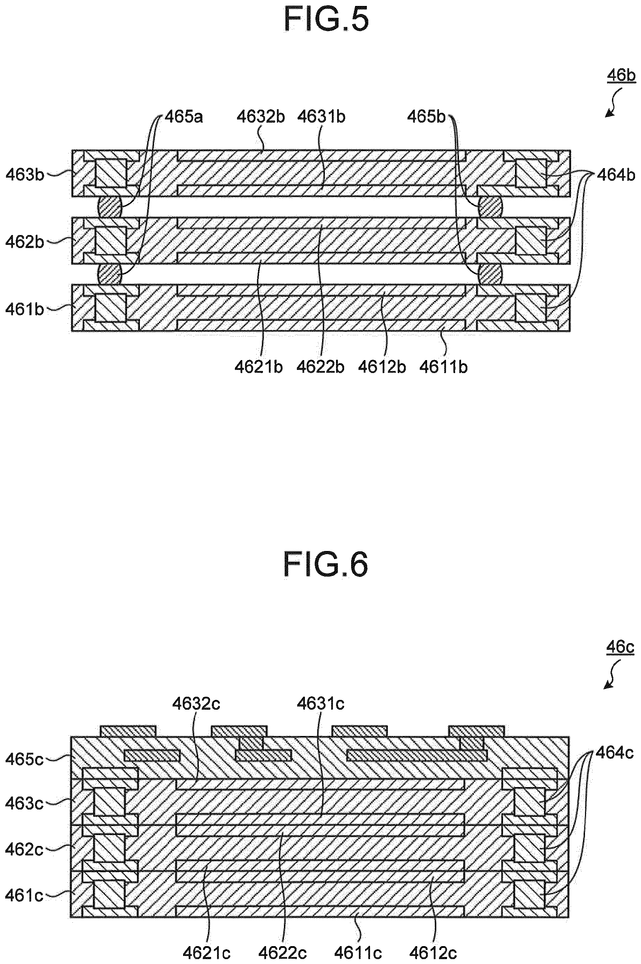

FIG. 5 is a cross-sectional view of the relay member according to the first modification of the embodiments of the present invention. A relay member 46b illustrated in FIG. 5 is formed by lamination of a plurality of silicon substrates 461b, 462b, and 463b. Planar type electronic devices 4611b, 4612b, 4621b, 4622b, 4631b, and 4632b are formed on both surfaces of the plurality of silicon substrates 461b, 462b, and 463b, respectively. Further, the plurality of silicon substrates 461b, 462b, and 463b is electrically connected to each other by through vias 464b and bumps 465a and 465b. The bump 465b may be disposed at a position different from a vertical direction of the through vias 464b to connect the silicon substrates 461b, 462b, and 463b, or may be disposed at the same position as the vertical direction of the through vias 464b to connect the silicon substrates 461b, 462b, and 463b. A resin layer (not illustrated) may be formed in each gap among the plurality of silicon substrates 461b, 462b, and 463b to reinforce connection strength among the silicon substrates. In addition, the electronic devices 4611b, 4612b, 4621b, 4622b, 4631b, and 4632b are any one of buffers, capacitors, inductors, and resistors that amplify the image signal generated by the image sensor 44 and output the amplified image signal to the signal cable 48.

According to the first modification of the embodiments of the present invention as described above, by formation of the planar type electronic devices 4611b, 4612b, 4621b, 4622b, 4631b, and 4632b on both surfaces of the silicon substrates 461b, 462b, and 463b, further miniaturization can be achieved.

Note that, in the first modification of the embodiments of the present invention, the planar type electronic devices 4611b, 4612b, 4621b, 4622b, 4631b, and 4632b are formed on both surfaces of the silicon substrates 461b, 462b, and 463b, respectively. However, for example, the plurality of planar type electronic devices may be formed in parallel on one surface of the silicon substrate 462b.

### (Second Modification)

Next, a second modification of the embodiments of the present invention will be described. The present second modification of the embodiments differs from the above-mentioned first and second embodiments only in a configuration of the relay member according to the first and second embodiments. Specifically, a relay member according to the present second modification of the embodiments is formed by further lamination of a multilayer wiring layer on the laminated silicon substrates. Hereinafter, the configuration of the relay member according to the present second modification of the embodiments will be described.

FIG. 6 is a cross-sectional view of the relay member according to the second modification of the embodiments of the present invention. A relay member 46c illustrated in FIG. 6 is formed by lamination of a plurality of silicon substrates 461c, 462c, and 463c. Furthermore, a multilayer wiring layer 465c is laminated and formed on an outermost layer of the silicon substrate 463c. Planar type electronic devices 4611c, 4612c, 4621c, 4622c, 4631c, and 4632c are formed on both surfaces of the plurality of silicon substrates 461c, 462c, and 463c, respectively. Furthermore, each of the plurality of silicon substrates 461c, 462c, and 463c and the multilayer wiring layer 465c is electrically connected by through vias 464c. In the present modification, each of the planar type electronic devices 4611c, 4612c, 4621c, 4622c, 4631c, and 4632c is connected by directly bonding the through vias 464c, without use of a bump. The electronic devices 4611c, 4612c, 4621c, 4622c, 4631c, and 4632c are any one of buffers, capacitors, inductors, and resistors that amplify the image signal generated by the image sensor 44 and output the amplified image signal to the signal cable 48.

A material capable of connecting an electronic component or a signal cable by soldering, for example, an electrode in which an Au plating layer is formed on a Cu layer via an Ni barrier layer, is formed on an outermost surface of the multilayer wiring layer 465c. With this configuration, another electronic component, passive element, and signal cable 48 can be connected by soldering. Note that a multilayer FPC substrate may be laminated as the multilayer wiring layer 465c, or the multilayer wiring layer 465c may be formed on the silicon substrate 463c by a well-known build-up method.

According to the second modification of the embodiments of the present invention described above, by lamination and formation of the multilayer wiring layer 465c on the outermost layer of the silicon substrate 463c, high-density wiring can be performed.

In addition, according to the second modification of the embodiments of the present invention, by lamination and formation of the multilayer wiring layer 465c on the outermost layer of the silicon substrate 463c, another electronic component, passive element, and signal cable 48 can be connected by soldering.

Furthermore, according to the second modification of the embodiments of the present invention, the planar type electronic devices 4611c, 4612c, 4621c, 4622c, 4631c, and 4632c are formed on both surfaces of the plurality of silicon substrates 461c, 462c, and 463c. With this configuration, further miniaturization can be achieved.

### (Third Modification)

Next, a third modification of the embodiments of the present invention will be described. The present third modification of the embodiments differs from the above-mentioned first embodiment only in a configuration of the imaging unit 40 according to the first embodiment. Specifically, an imaging unit according to the present third modification of the embodiments is configured by use of an image sensor (imager chip) of a front illumination type (Front Side Illumination), and a relay unit is laminated on a back surface of the image sensor. Hereinafter, a configuration of the imaging unit according to the present third modification of the embodiments will be described.

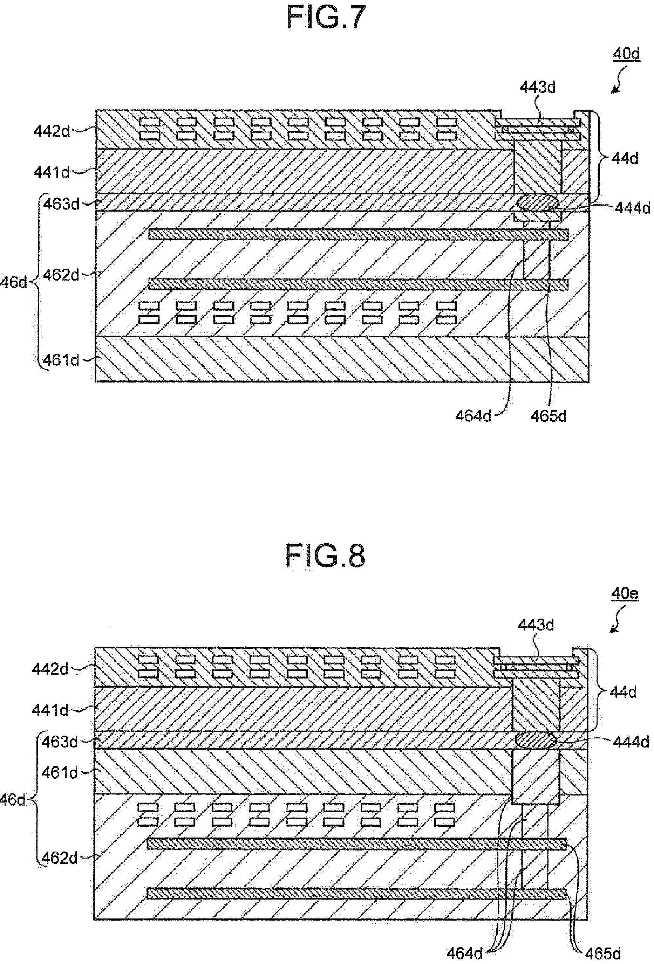

FIG. 7 is a schematic cross-sectional view of the imaging unit according to the third modification of the embodiments of the present invention. An imaging unit 40d illustrated in FIG. 7 includes an image sensor 44d that generates an image signal (electric signal) by receiving light and performing photoelectric conversion, and a relay member 46d that relays the image sensor 44d and the signal cable 48.

The image sensor 44d includes a semiconductor substrate 441d on which a light receiving unit (pixel unit) in which a plurality of pixels (photodiodes) is arrayed in a two-dimensional matrix is formed, the light receiving unit outputting an electric signal by receiving light and performing photoelectric conversion, a wiring layer 442d laminated on the semiconductor substrate 441d, and a through via 464d.

The relay member 46d includes a semiconductor substrate 461d (silicon substrate) on which a circuit and the like are formed, an electronic device layer 462d formed by lamination of a dielectric and the like on the semiconductor substrate 461d, and a connecting portion 463d provided on an outermost layer of the electronic device layer 462d and connected to the image sensor 44d. The electronic device layer 462d is either a buffer that amplifies and outputs the image signal output from the image sensor 44d, or a bypass capacitor that flows an AC component such as noise to the ground. The electronic device layer 462d includes electrodes 465d. The electrodes 465d are electrically connected to a through via 443d via the through via 464d and a bump 444d.

According to the third modification of the embodiments of the present invention described above, the relay member 46d is provided on the back surface side of the image sensor 44d. With this configuration, further miniaturization can be achieved.

In addition, in the third modification of the embodiments of the present invention, a back surface side of the semiconductor substrate 441d of the image sensor 44d and a back surface side of the semiconductor substrate 461d of the relay member 46d may be connected and laminated. As illustrated in FIG. 8, in an imaging unit 40e, the semiconductor substrate 461d is electrically connected to the semiconductor substrate 441d via the bump 444d and the through via 443d. With this configuration, by providing the relay member 46d on the back surface side of the image sensor 44d, further miniaturization can be achieved.

### (Fourth Modification)

Next, a fourth modification of the embodiments of the present invention will be described. The present fourth modification of the embodiments differs from the above-mentioned first embodiment only in a configuration of the imaging unit 40 according to the first embodiment. Specifically, an imaging unit according to the present fourth modification of the embodiments is configured by use of an image sensor (imager chip) of a back illumination type (Back Side Illumination), and a relay unit is laminated on a back surface of the image sensor. Hereinafter, a configuration of the imaging unit according to the present fourth modification of the embodiments will be described.

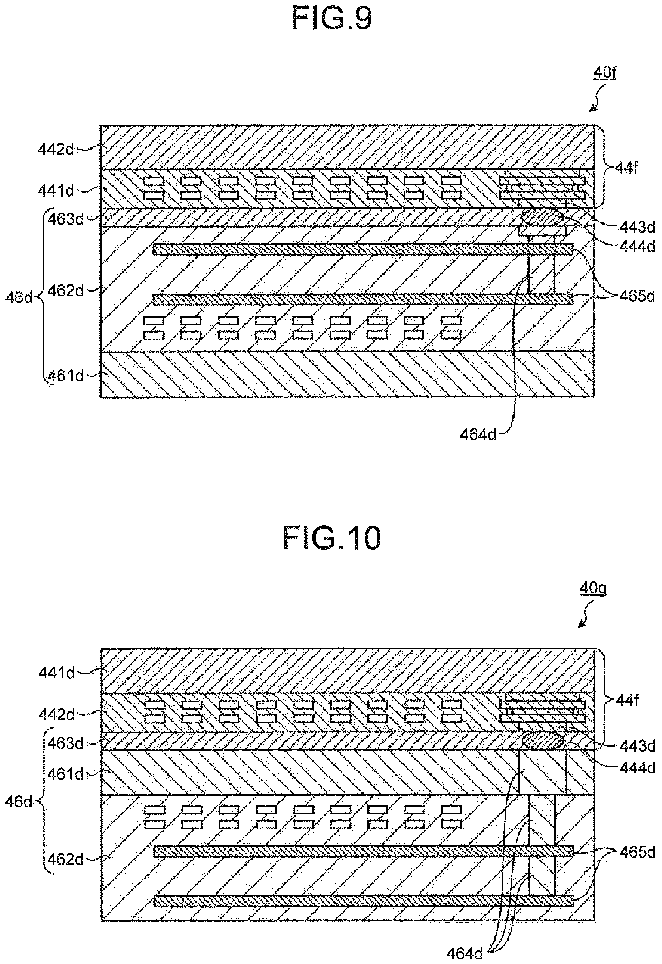

FIG. 9 is a schematic cross-sectional view of the imaging unit according to the fourth modification of the embodiments of the present invention. An imaging unit 40f illustrated in FIG. 9 includes an image sensor 44f that generates an image signal (electric signal) by receiving light and performing photoelectric conversion, and the relay member 46d according to the above-mentioned third modification of the embodiment.

The image sensor 44f includes the semiconductor substrate 441d on which the light receiving unit (pixel unit) in which the plurality of pixels (photodiodes) is arrayed in the two-dimensional matrix is formed, the light receiving unit outputting the electric signal by receiving light and performing photoelectric conversion, the wiring layer 442d laminated on the semiconductor substrate 441d, and the through via 443d. The image sensor 44f is electrically connected to the relay member 46d via the through via 443d and the bump 444d.

According to the fourth modification of the embodiments of the present invention described above, the relay member 46d is laminated on the back surface of the image sensor 44d. With this configuration, further miniaturization of the imaging unit 40f can be achieved.

In addition, in the fourth modification of the embodiments of the present invention, a front surface side of a light receiving unit 442d of the image sensor 44f and the back surface side of the semiconductor substrate 461d of the relay member 46d may be connected and laminated. As illustrated in FIG. 10, in an imaging unit 40g, the front surface side of the light receiving unit 442d (wiring layer) of the image sensor 44f and the back surface side of the semiconductor substrate 461d of the relay member 46d are electrically connected via the bump 444d. With this configuration, further miniaturization can be achieved.

As described above, the present invention may include various embodiments not described here, and it is possible to make various design changes and the like within the scope of the technical idea specified by the claims. Reference Signs List

1 ENDOSCOPE SYSTEM
2 ENDOSCOPE
3 UNIVERSAL CORD
3A DISTAL END PORTION
3B BENDING PORTION
3C FLEXIBLE TUBE PORTION
4 OPERATING UNIT
4a TREATMENT INSTRUMENT INSERTION PORT
4b BENDING OPERATION KNOB
5 CONNECTOR PORTION
6 PROCESSOR
7 DISPLAY DEVICE
8 LIGHT SOURCE DEVICE
30 INSERTION PORTION
35 IMAGING DEVICE
40, 40a, 40d, 40e IMAGING UNIT
41 DISTAL END PORTION MAIN BODY
41a ADHESIVE
42 CLADDING TUBE
43 LENS UNIT
43b LENS HOLDER
44, 44d, 44f IMAGE SENSOR
45, 45a FLEXIBLE PRINTED BOARD
46, 46a, 46b, 46c, 46d RELAY MEMBER
47 ELECTRIC CABLE BUNDLE
48 SIGNAL CABLE
49 GLASS SLID
50 HEAT SHRINKABLE TUBE
51 ADHESIVE RESIN
82 BENDING WIRE
100 PASSIVE ELEMENT
441, 442d LIGHT RECEIVING UNIT
442, 441d SEMICONDUCTOR SUBSTRATE
443, 464, 464a, 464b, 464c, 464d THROUGH VIA
444, 444d BUMP
461 to 463, 461a to 463a SILICON SUBSTRATE
461d SEMICONDUCTOR SUBSTRATE
465c MULTILAYER WIRING LAYER
465d ELECTRODE
4611, 4621, 4631, 4611b, 4611c, 4621b ELECTRONIC DEVICE

## Claims

1. An imaging unit, comprising:
an image sensor configured to generate an image signal by receiving light and performing photoelectric conversion; and
a relay member including a silicon substrate provided on a back surface side of the image sensor opposite to a light receiving surface of the image sensor, a planar type electronic device being formed on the silicon substrate, and relay the image sensor and a signal cable that transmits the image signal.

2. The imaging unit according to claim 1, wherein a plurality of silicon substrates are laminated in the relay member.

3. The imaging unit according to claim 2, wherein the plurality of silicon substrates are laminated in a direction orthogonal to an extending direction of the signal cable.

4. The imaging unit according to claim 2, wherein
the relay member further includes a flexible printed board that extends in parallel to an extending direction of the signal cable, and
the plurality of silicon substrates are laminated on the flexible printed board.

5. The imaging unit according to claim 2, wherein the plurality of silicon substrates are laminated in a direction parallel to an extending direction of the signal cable.

6. The imaging unit according to claim 5, wherein an area of each of the plurality of silicon substrates is equal to or less than a projected area when the image sensor is projected in the extending direction of the signal cable.

7. The imaging unit according to claim 2, wherein the plurality of silicon substrates are connected by a through via that passes through at least an adjacent silicon substrate.

8. The imaging unit according to claim 2, wherein the electronic device is formed on each one of both surfaces of each of the plurality of silicon substrates.

9. The imaging unit according to claim 1, wherein the electronic device is at least any one of a buffer, a capacitor, an inductor and a resistor.

10. An endoscope, comprising:
the imaging unit according to claim 1; and
an insertion portion that includes a cylindrical distal end portion formed of a hard member and is insertable into a subject,
wherein the insertion portion includes the imaging unit in an internal space of the distal end portion.
